# EUROPEAN PATENT SPECIFICATION

(11) **EP 3 829 862 B1**
(45) Date of publication and mention of the grant of the patent: **27.08.2025**
(21) Application number: 19843533.1
(22) Date of filing: 29.07.2019
(51) Int. Cl.: B32B 5/02, B32B 5/24, B32B 3/14, A47G 9/00

(54) **ABSORBENT MATERIAL AND METHOD OF MANUFACTURE THEREOF**
SAUGFÄHIGES MATERIAL UND VERFAHREN ZUR HERSTELLUNG DAVON
MATÉRIAU ABSORBANT ET SON PROCÉDÉ DE FABRICATION

(30) Priority: 30.07.2018 SG 10201806497T
(43) Date of publication of application: 09.06.2021
(73) Proprietor: Kisodo Pte. Ltd., Singapore 409051 (SG)
(72) Inventor: CHU, Ting-Man, Timothy, Singapore 409051 (SG)
(74) Representative: Mewburn Ellis LLP
(86) International application number: PCT/SG2019/050370
(87) International publication number: WO 2020/027726

(56) References cited:
- EP-A1- 1 870 065
- WO-A1-2014/152227
- CN-A- 103 290 615
- CN-U- 201 879 914
- CN-U- 205 467 700
- JP-A- 2011 224 267
- US-A- 4 377 615
- US-A- 5 374 259
- US-A- 5 476 456
- US-A- 5 901 706
- US-A1- 2016 067 118
- US-A1- 2016 145 781

## Description

### TECHNICAL FIELD OF THE INVENTION

The invention relates to an absorbent material, bed sheet made of the absorbent material and a method of manufacture thereof.

### BACKGROUND OF THE INVENTION

The following discussion of the background to the invention is intended to facilitate understanding of the present invention. However, it should be appreciated that the discussion is not an acknowledgment or admission that any of the material referred to was published, known or a part of the common general knowledge in any jurisdiction as at the priority date of the application. Disposable absorbent materials that are able to absorb liquids such as bodily fluids are useful in/for various applications. As an example application, adult diapers designed for a person with a body larger than that of an infant or toddler may be used by adults with various conditions, such as incontinence, mobility impairment, severe diarrhea or dementia. Consequently, adult diapers prevent structures such as beds and sofas from being stained. Adult diapers may be made in various forms, including those resembling traditional child diapers, underpants, and pads resembling sanitary napkins. However, usage of adult diapers can lead to negative emotions such as embarrassment and depression because of the stigma associated with adult diapers.

Current disposable absorbent materials may be made from fibrous substances that make these absorbent materials fluffy and bulky. As such, it may be difficult to transport, store and/or dispose of such absorbent materials. Consequently, there may be increased costs associated with manufacturing, transporting, storing and disposing of such absorbent materials.

Furthermore, existing disposable absorbent materials may be thick and non-breathable. Consequently, users may have an increased likelihood to suffer from bedsores.

WO2014/152227A1 discloses disposable sheet with an absorbent pad comprising multiple layers.

EP1870065A1 discloses an absorbent article comprising an absorbent member intervening between a liquid-permeable surface sheet and a backside sheet.

In light of the above, there exists a need to develop an absorbent material that ameliorates or overcomes the above disadvantages.

### SUMMARY OF THE INVENTION

In an aspect of the present disclosure, there is provided an absorbent material including: a first layer comprising a hydrophilic non-woven fabric; and a second layer comprising a hydrophobic film, the second layer arranged adjacent to the first layer; wherein the first layer is adapted to allow a first fluid and a second fluid to pass through; and wherein the second layer is adapted to allow the first fluid to pass through and is adapted to minimize or block the second fluid from passing through.

In various aspects, the hydrophilic non-woven fabric comprises a polymer.

In various aspects, the polymer is polypropylene or polyethylene.

In various aspects, the hydrophobic film comprises polyethylene.

In various aspects, the hydrophilic non-woven fabric consists of polypropylene and the hydrophobic film consists of polyethylene.

In various aspects, the thickness of the absorbent material is 0.1 millimetres (mm) to 5 mm.

The second layer is laminated adjacent to the first layer.

The first layer is made using a thermal bonding process.

The first layer comprises short fibers.

In various aspects, the short fibers have an average length of 38 mm to 51 mm. The first fluid is air and the second fluid is a liquid.

The absorbent material consists of the first layer and the second layer.

In various aspects, the first layer is made of hydrophilic non-woven fabric consisting of polypropylene and the second layer is made of hydrophobic film consisting of polyethylene.

In various aspects, the absorbent material further comprises a super absorbent polymer.

In various aspects, the super absorbent polymer is integrated with the first layer or integrated with the second layer.

In various aspects, the first layer or the second layer is added with biodegradable additives.

In another aspect of the present disclosure, there is provided a bed sheet made of an absorbent material as described above.

In various aspects, the absorbent material of the bed sheet is disposable.

In another aspect of the present disclosure, there is provided a method of manufacturing an absorbent material, the method comprising: the step of thermal bonding to form a first layer comprising a hydrophilic non-woven fabric; the step of laminating a second layer comprising a hydrophobic film adjacent to the first layer; wherein the first layer is adapted to allow a first fluid and a second fluid to pass through; and wherein the second layer is adapted to allow the first fluid to pass through and is adapted to minimize or block the second fluid from passing through.

In various aspects, the step of laminating the second layer adjacent to the first layer comprises heat, mechanical or chemical means.

In various aspects, the step of thermal bonding comprises the step of carding.

In various aspects, the hydrophilic non-woven fabric consists of polypropylene and the hydrophobic film consists of polyethylene.

In various aspects, the absorbent material consists of the first layer and the second layer.

In various aspects, the step of thermal bonding to form the first layer occurs at a temperature of 150°C to 180°C.

In various aspects, the method further comprises the step of manufacturing fibers for the hydrophilic non-woven fabric.

In various aspects, the step of manufacturing fibers for the hydrophilic non-woven fabric comprises the step of crimping.

In various aspects, the step of crimping occurs at a pressure of 0.1 bar to 5.0 bar.

In various aspects, the step of crimping occurs at a temperature of 60°C to 120°C.

Other aspects of the disclosure will become apparent to those of ordinary skill in the art upon review of the following description of specific aspects of the disclosure in conjunction with the accompanying drawings.

The invention is defined in the claims.

### BRIEF DESCRIPTION OF THE DRAWINGS

The disclosure will now be described, by way of example only, with reference to the accompanying drawings, in which:
Figure 1 illustrates a planar view of an absorbent material in accordance with an aspect of the disclosure;
Figure 2 illustrates a cross-sectional view of the absorbent material of Figure 1;
Figure 3 illustrates a process of manufacturing fibers for a non-woven fabric in accordance with another aspect of the disclosure; and
Figure 4 illustrates a process of manufacturing a non-woven fabric using fibers made from the process of Figure 3 in accordance with another aspect of the disclosure.

Other arrangements of the disclosure are possible and, consequently, the accompanying drawings are not to be understood as superseding the generality of the preceding description of the disclosure.

### DETAILED DESCRIPTION

Particular aspects of the present disclosure will now be described with reference to the accompanying drawings. The terminology used herein is for the purpose of describing particular aspects only and is not intended to limit the scope of the present disclosure. Other definitions for selected terms used herein may be found within the detailed description of the disclosure and apply throughout the description. Additionally, unless defined otherwise, all technical and scientific terms used herein have the same meanings as commonly understood by one or ordinary skill in the art to which the present disclosure belongs. Where possible, the same reference numerals are used throughout the figures for clarity and consistency.

Throughout the specification, unless the context requires otherwise, the word "comprise" or variations such as "comprises" or "comprising", will be understood to imply the inclusion of a stated integer or group of integers but not the exclusion of any other integer or group of integers.

Throughout the specification, unless the context requires otherwise, the word "include" or variations such as "includes" or "including", will be understood to imply the inclusion of a stated integer or group of integers but not the exclusion of any other integer or group of integers.

Throughout the specification, unless the context requires otherwise, the term "fluid" includes but is not limited to a liquid such as water and bodily fluid(s). The bodily fluid(s) may include blood, urine, and a combination thereof. The fluid may include one or more gas(es) and can include air which is a mixture of gases.

Throughout the specification, unless the context requires otherwise, the term "breathable" refers to the ability of the absorbent material or a layer thereof to allow air and/or water vapour to pass through.

Throughout the specification, unless the context requires otherwise, the term "flexible" refers to the ability to be folded for storage and/or transportation purposes, but rigid enough to resist tear when the absorbent material is in use.

Throughout the specification, unless the context requires otherwise, the term "durable" refers to the ability of the absorbent material to retain its original shape and size under application of an external force, and to resist wear and tear. In the context of the disclosure, the term "durable" refers to the ability of the absorbent material to resist being torn apart when subjected to a position or weight, such as a position of the adult when lying down on a bed or the weight of an adult.

In accordance with an aspect of the disclosure or disclosure there is an absorbent material including a first layer made of a hydrophilic non-woven fabric; and a second layer made of a hydrophobic film, the second layer arranged adjacent to the first layer; wherein the first layer is adapted to allow a first fluid and a second fluid to pass through; and wherein the second layer is adapted to allow the first fluid to pass through and is adapted to minimize or block the second fluid from passing through.

With the arrangement and selection of the material used for the first layer, the first layer is permeable to the first fluid. The first fluid may be air which is able to pass through the first layer, contact the second layer and pass through the second layer. As such, the first layer and the second layer of the absorbent material are permeable to the first fluid. In addition, the first layer is permeable to the second fluid. The second fluid may be a liquid such as water, urine, blood or a combination thereof is able to pass through the first layer, contact the second layer and then be minimized or blocked from passing through the second layer. As such, the second layer differs from the first layer in that the second layer is adapted to allow the first fluid to pass through and is adapted to minimize or block the second fluid from passing through. In various aspects, the second fluid passes through the first layer via capillary action which will be described in further detail.

The absorbent material consists only of two layers, i.e. the first layer and the second layer. As such, there is no need for any additional layer(s) disposed between the first layer and the second layer or adjacent to the first layer or adjacent to the second layer. Advantageously, the absorbent material may be relatively thin, light weight and relatively easy to transport, store and/or dispose of. Consequently, the absorbent material is relatively cheap, easy to manufacture and desirable for use as a disposable absorbent material.

Notwithstanding the above, various aspects may also contribute to making the absorbent material relatively cheap, easy to manufacture and desirable for use as a disposable material. As such, it would be appreciated by a person skilled in the art that an important consideration by the inventors of the present disclosure is affordability of the absorbent material for the intended application(s).

In contrast, prior art absorbent materials typically consists of more than two layers and is therefore relatively more expensive to manufacture.

In various aspects, the absorbent material further comprises at least one additive such as a super absorbent polymer or biodegradable additive. In some aspects, the additive can be integrated with the first layer. In some aspects, the additive can be integrated with the second layer. In some aspects, the super absorbent polymer may be applied to the first layer and/or the second layer by spraying. As such, the super absorbent polymer may be in the form of small beads. In an aspect, the absorbent material does not comprise of or contain any additive. Consequently, the absorbent material is relatively cheap, easy to manufacture and desirable for use as a disposable material.

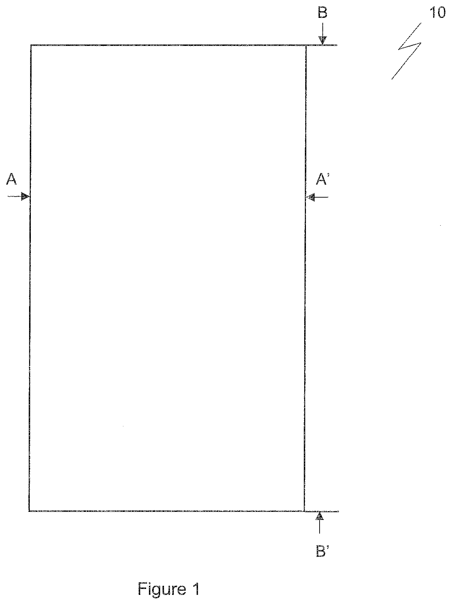

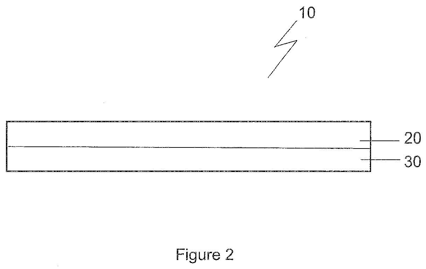

With reference to Figure 1 and Figure 2, the absorbent material 10 comprises a first layer 20 made of/ formed of a hydrophilic non-woven fabric; a second layer 30 adjacent to the first layer 20; wherein the second layer 30 is made of/ formed of a hydrophobic film. As may be appreciated, the non-woven fabric is hydrophilic and may therefore allow a fluid such as urine, blood, water vapour, and/or water to pass through.

In some aspects, the absorbent material 10 is breathable. This is at least because the first layer 20 and the second layer 30 are both breathable. At least a part of the first layer 20 and at least a part of the second layer 30 are breathable, although it is more advantageous that the entire first layer 20 and the entire part of the second layer 30 are both breathable. The first layer 20 comprises the hydrophilic non-woven fabric which appreciably is breathable.

It is appreciable that as the non-woven fabric first layer 20 is hydrophilic, it is capable of wicking away a fluid from the user when the first layer 20 is in contact with the user. In other words, there is a wicking effect. In particular, the wicking effect is caused by movement of the fluid away from the user via capillary action. As such, the first layer 20 may minimize or eliminate wetness of the user because there is no or minimal build-up of the fluid. Presence of the fluid may facilitate the growth of bacteria and/or mould. Consequently, use of the absorbent material 10 may be relatively more comfortable and hygienic compared to currently known absorbent materials, as will be explained below.

In contrast, prior art absorbent materials may have a first layer that is made of a hydrophobic non-woven fabric, which would not wick away a fluid from the user when the first layer is in contact with the user. Furthermore, some prior art absorbent materials may allow air to pass through but are not capable of wicking away a fluid from the user, whereby the fluid is a liquid such as urine, blood and/or water. As such, prior art absorbent materials may be uncomfortable and unhygienic to the user because a portion of the user may be wet due to the fluid and possibly remain wet. Consequently, growth of bacteria and/or mould may be facilitated due to build-up of the fluid.

In some aspects, the absorbent material 10 may be cheap to manufacture and comfortable to the user because of the material used for the first layer 20 and/or the second layer 30. Furthermore, the specific combination of the material used for the first layer 20 and the second layer 30 may be important and lead to one or more advantages.

In various aspects, the non-woven fabric is made of a polymer. In some aspects, the polymer is polypropylene (PP) or polyethylene (PE) or a combination thereof. In some aspects, the non-woven fabric may be predominantly polypropylene. In some aspects, the non-woven fabric consists of polypropylene. In other words, the non-woven fabric does not contain polyethylene or a polyester. As such, the non-woven fabric may be used in bulk quantities because polypropylene is relatively cheap compared to other materials such as polyethylene. The use of polypropylene is particularly suitable for the first layer 20 because it is substantially inert towards a wide range of chemicals. In addition, polypropylene is more inert towards chemicals and organic solvents compared to polyethylene. Consequently, the non-woven fabric of the present disclosure may be attractive to the user because it may be comfortable to the user. For instance, there may be no or negligible skin irritation if a part of the user comes into contact with the non-woven fabric.

In various aspects, the non-woven fabric is smooth and soft to the touch. At the same time, there is sufficient rigidity such that the non-woven fabric may retain its integrity, such that it is capable of not being torn apart when subjected to a weight, such as the weight of an adult, whereby the adult may be laying down. Consequently, the use of the non-woven fabric may make it attractive to the user because it may be comfortable to the user.

As mentioned above, the second layer 30 is made of/formed of a hydrophobic film which allows a first fluid (e.g. air) to pass through but is impermeable to a second fluid (e.g. water, urine, blood) etc. In various aspects, the hydrophobic film is made of a polymer. In some aspects, the polymer is polyester, polyethylene, polyurethane or a combination thereof. In some aspects, the hydrophobic film may be predominantly polyethylene. In some aspects, the hydrophobic film consists of polyethylene.

In various aspects, the hydrophobic film may be durable and flexible. When a durable hydrophobic film is used, the absorbent material 10 would not easily tear when it comes into contact with a liquid. When a flexible hydrophobic film is used, the absorbent material 10 may be easily folded to reduce the surface area, thereby making it easier for transportation and/or storage. As the hydrophobic film may be durable and flexible, the absorbent material 10 may be folded without the formation of creases i.e. the absorbent material 10 may be in a compact position without the formation of creases. Consequently, the absorbent material 10 may be folded for storage purposes when not in use. Conversely, the absorbent material 10 may be unfolded without the formation of undesired creases when in use.

In various aspects, the hydrophobic film may be soft. In various aspects, negligible sound will be produced when the user moves.

In various aspects, the first layer 20 is made of a different material from the second layer 30. As the first layer 20 is hydrophilic and the second layer 30 is hydrophobic, the first layer 20 wicks away a fluid from the user and the second layer 30 functions as a barrier for preventing the fluid from wetting a surface adjacent to the second layer 30. The surface may be part of a structure such as a bed. Consequently, the first layer 20 and the second layer 30 synergistically work together in minimizing or eliminating wetness of the user and the surface adjacent to the second layer 30. In some aspects, the first layer 20 is predominantly made of polypropylene and the second layer 30 is predominantly made of polyethylene. In some aspects, the first layer 20 consists of polypropylene and the second layer 30 consists of polyethylene.

The second layer 30 is laminated adjacent to the first layer 20. Advantageously, the first layer 20 and the second layer 30 are substantially integrated or bonded to each other, such that it is difficult if not impossible to separate the two layers from each other, particularly when in use. As such, if the absorbent material 10 comes into contact with a liquid, the liquid would not be able to cause the two layers to separate, thereby maintaining the integrity of the absorbent material 10. The step of laminating may make use of heat, mechanical or chemical means.

The first layer 20 comprises short fibers. Short fibers allows movement of a fluid from the user via capillary action. In other words, there is a wicking effect. As such, the first layer 20 may minimize or eliminate wetness of the user. The short fibers of the first layer 20 have an average length of about 30 millimeters (mm) to about 60 mm.

In various aspects, the first layer 20 and/or second layer 30 may be coloured to provide an attractive colour to attract the attention of a user. The first layer 20 and/or second layer 30 may be coloured with a dye, wherein the colour of the dye is at least one colour selected from red, blue, green, yellow, violet, orange, black, gold, silver, grey or white.

In various aspects, the first layer 20 and/or second layer 30 may have prints such as words or images on it. The prints may advantageously enhance the visual appearance and attractiveness of absorbent material 10 because the prints may be visually appealing words or images. The prints may be opaque, partially opaque or translucent. The presence of prints may be useful when the target audience is adults because the prints may help reduce negative emotions such as embarrassment and depression when used in various applications, including, but not limited to, adult diapers. In some aspects, the prints may be a wet indicator that reacts when exposed to a fluid such as a liquid, thereby indicating that the absorbent material 10 or a part thereof is wet.

Depending on the dimensions of the absorbent material 10, the absorbent material 10 may be applied to various applications including, but not limited to, sanitary napkins, diapers for children, diapers for adults that may be made in various forms, including those resembling traditional child diapers, underpants, and pads resembling sanitary napkins, protective covers for structures, such as but not limited to household furniture, and protective clothing, such as but not limited to the medical field. Examples include bed sheets, table cloths, covers for pet cages, isolation gowns, surgical gowns, surgical drapes and covers, surgical masks, surgical scrub suits and caps, and medical packaging. In a preferred aspect, the absorbent material 10 is used to make protective covers for bedding, such as bed sheets. In another preferred aspect, the absorbent material 10 is intended for disposable use, which may be one-time or single use.

In various aspects, the thickness of absorbent material 10 may range from about 0.1 mm to about 5 mm, about 0.2 mm to about 5 mm, about 0.5 mm to about 5 mm, about 0.5 mm to about 4 mm, about 1 mm to about 5 mm, about 1 mm to about 4 mm, about 0.1 mm to about 0.5 mm or about 0.5 mm to about 2 mm. In a preferred aspect, the thickness of the absorbent material 10 may be about 0.2 mm to about 0.3 mm. As such, the absorbent material 10 is relatively thin and lightweight, thereby making it easier to transport, store and dispose compared to thicker and bulkier absorbent materials currently known.

One or more dimensions of the absorbent material 10, such as a height, a length, or a width of the absorbent material 10 may be adjusted according to the application. Advantageously, as the absorbent material 10 is made of a relatively cheap material, adjusting the height and width of the absorbent material 10 does not substantially increase the manufacturing costs.

In various aspects, the height of the absorbent material 10 may range from about 180 centimeters (cm) to about 210 cm so that the absorbent material 10 may cover, such as but not limited to, a single size bed, a queen size bed, a king size bed or a super king size bed. In various aspects, the width of the absorbent material 10 may range from about 90 cm to about 210 cm so that the absorbent material 10 may cover, such as but not limited to, a single size bed, a queen size bed, a king size bed or a super king size bed.

It is appreciable that the first layer 20 and the second layer 30 of the absorbent material 10 work in tandem to achieve one or more of the following results:-
(i.) wicking effect of the first layer 20 next to or directly adjacent to skin for dryness and comfort and hydrophobic breathable film of the second layer 30 to minimize/prevent liquid leakage but the second layer 30 is permeable to air;
(ii.) the second layer 30 reinforces the structural integrity of the absorbent material 10 by minimizing the tearing of the first layer 20 when the first layer 20 comes into contact with a liquid such as urine, blood, water etc.;
(iii.) any moisture will be wicked onto the hydrophobic second layer 30, which prevents leakage of the liquid to a structure such as a mattress.

In accordance with another aspect of the disclosure or disclosure there is a bed sheet comprising an absorbent material as described above. Advantageously, bedsores may be minimized or prevented because the absorbent material is breathable. Furthermore, the bed sheet may be relatively cheap, easy to manufacture and desirable for disposability.

In accordance with another aspect of the disclosure or disclosure there is a method of manufacturing an absorbent material comprising the step of thermal bonding to form a first layer comprising a hydrophilic non-woven fabric; and a step of arranging a second layer comprising a hydrophobic film adjacent to the first layer; wherein the first layer is adapted to allow a first fluid and a second fluid to pass through; wherein the second layer is adapted to allow the first fluid to pass through and is adapted to minimize or block the second fluid from passing through.

In various aspects, the method further comprises the step of providing the second layer comprising a hydrophobic film.

In various aspects, the properties of the absorbent material such as strength, thickness, drape and softness may be altered depending on the method of manufacturing used so as to achieve desirable properties. The desirable properties may be selected based on the type of application(s) relating to the use of the absorbent material. In an aspect, the absorbent material is used to make protective covers for structures, such as bed sheets.

In various aspects, the step of thermal bonding makes use of short fibers to form the first layer. In contrast, spun bonding makes use of continuous fibers to form the first layer. Advantageously, the presence of short fibers in the first layer allows movement of a fluid from the user via capillary action. In other words, short fibers produce or facilitate a wicking effect. As such, the first layer may minimize or eliminate wetness of the user.

In various aspects, the step of thermal bonding may advantageously lead to an appropriate thickness, sufficient strength, drape and softness of the absorbent material at a relatively low cost. Advantageously, the cost of mass production may be relatively low, thereby making it relatively affordable for use as a disposable absorbent material. In contrast, other methods such as adhesive bonding may generate sufficient strength but cause stiffness of the absorbent material because of the presence of an adhesive, thereby lowering the drape and softness of the absorbent material.

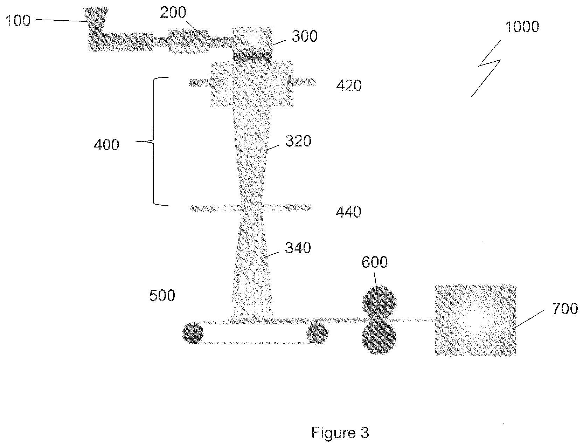

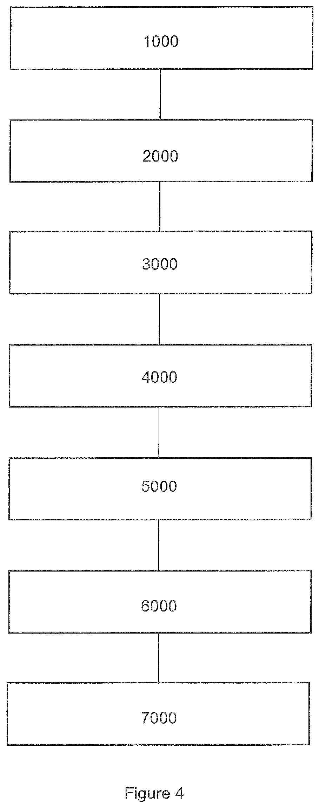

In various aspects and as illustrated in Figure 3 and Figure 4, the method of manufacturing an absorbent material comprises the step of manufacturing staple fibers or fibers for a non-woven fabric (step 1000). Accordingly, a first control panel is provided to set parameters for the production of fibers from polymer chips, such as PP chips. In some aspects, the PP chips have a unit melt flow rate (230°C, 2.16 kg) of about 20 to about 35 grams per 10 min (g/10min), preferably about 16 to about 17 g/10min. In various aspects and as illustrated in Figure 3, the polymer for the first layer 20 is melted in an extruder 100 to form a molten material, and the molten material is transferred to a die block 300 via a pump such as spin pump 200, thereby forming fibers 320. The control panel sets the temperature for the PP chips to melt in the extruder 100, thereby forming thin strands of fibers 320, which then exit through at least one spinneret (not illustrated). In addition, the control panel ensures that the melting point and the melt flow rate of the PP chips entering the extruder 100 are correct. Although the polymer starting material may come in the form of pellets or other particulate form and melted to a liquid (which can be in a pumpable state), other fiber-forming liquids such as a polymer solution could also be used.

In various aspects, fibers 320 which are semi-processed, are transferred to a stretching chamber or a first stretch roller 400, wherein a stream of air 420 or another gas for cooling is ejected to simultaneously draw down and cool the fibers 320 as the fibers 320 pass through the stretching chamber 400. In various aspects, the stream of air or another gas may be heated to obtain a desired temperature of the extruded fibers 320 and/or to facilitate drawing of the fibers 320. In a preferred aspect, the fibers 320 may be passed through a steam box, so that the fibers 320 may be subjected to steam before crimping. In other words, the fibers 320 may be conditioned by steam prior to passing through a crimper machine 600.

In various aspects, the fibers 320 may optionally be passed through a second stretch roller (not illustrated).

In various aspects, the fibers 320 may be subjected to a stream of oil 440 (i.e. spin finished), which may assist in crimping the fibers 320 so as to form a mass of fibers 320 that may or may not be coherent and take the form of a tow 340.

In various aspects, after passing through the stretching chamber 400, but prior to collection, the mass of fibers 320 may be subjected to a number of additional processing steps. Upon collection, the mass of fibers 320 may be subjected to one or more apparatus such as but not limited to a bonding oven, calender, hydroentangling mechanical bonder, embossing station, laminator, cutter. In some aspects, air-through bonding is not used in the present disclosure.

In various aspects, after passing through the stretching chamber 400, the tow 340 may be laid down on a belt 500 so that the tow 340 may be passed through a crimper machine or crimper 600.

In various aspects, a second control panel is provided to control the stretching chamber 400, and the second stretch roller if present, and guide the fibers 320 to the crimper machine 600. The second control panel also controls the crimper machine 600. As such, when the fibers 320 pass through the crimper machine 600, the crimper machine 600 will press the fibers 320 so that they become crimp and form crimped fibers 320. The step of crimping occurs at a pressure of about 0.1 bar to about 5.0 bar and at a temperature of about 60°C to about 120°C. Advantageously, crimping creates a softer fabric by reducing the "straightness" of the fibers 320, between bond points created in the thermal bonding step, as well as fiber-to-fiber bonds. More advantageously, crimping leads to fibers 320 having better strength.

In various aspects and as illustrated in Figure 3, the crimped fibers 320 are passed through a dryer 700 to dry or anneal the crimped fibers 320. At the same time, the fibers 320 will be cooled by passing through at least one roller to dry the fibers 320, such as by air. Consequently, any oil, moisture and/or undesired substance may be removed from the fibers 320 and the fibers 320 are cooled to a suitable temperature for further processing. After the fibers 320 are dried, they are passed through a cutter box (not illustrated), which will cut the fibers 320 into a desired length, such as a length required by a customer. In various aspects, the fibers 320 are short and may have an average length of about 30 mm to about 60 mm, about 35 mm to about 55 mm or about 38 mm to about 51 mm.

In various aspects, the cut fibers 320 are optionally transported to a baler machine, whereby the transporting may be carried out using an air system. The baler machine compresses the cut fibers 320 into a compact form so that the fibers 320 are more suitable for packing. Subsequently, the fibers 320 are packed using PP strapping bands to form bales of fibers 320 and sent to a warehouse to set or relax the fibers 320 for a period of time, such as at least 48 hours, preferably for one week or two weeks.

In various aspects and as illustrated in Figure 4, the method of manufacturing the absorbent material comprises the step of manufacturing the non-woven fabric. The non-woven fabric is made using thermal bonding, which is described in steps 2000, 3000, 4000, 5000, 6000 and 7000.

In various aspects, a first bale of fibers 320 may be opened and placed inside at least one fiber opener to loosen the fibers 320 so that the fibers 320 are at least partially loosened. Subsequently, the at least partially loosened fibers 320 are transferred from the first fiber opener to a second fiber opener, which will further loosen the fibers 320. If necessary, the loosened fibers 320 will be transferred to a third fiber opener, which comprises a control unit for determining how much fibers 320 to receive from the second fiber opener. Similar to the first fiber opener and the second fiber opener, the third fiber opener is configured to further loosen the fibers 320.

In various aspects and as illustrated in Figure 4, the method may further comprise the step of passing the fibers 320 through a carding feeder such as a FBK unit manufactured by Trützschler GmbH & Co. KG (step 2000). The fibers 320 may be transferred from the cutter box or a fiber opener (such as the first fiber opener, the second fiber opener or the third fiber opener) to the FBK unit. The primary function of the FBK unit is to bring the loosened fibers 320 to a kiss roller machine which comprises of a plurality of carding rollers. Importantly, the FBK unit regulates or controls the flow of an amount of fibers 320 to the carding rollers. For example, if the production of 16 grams of non-woven fabric is desired, the FBK unit will release an amount of fibers 320 that corresponds to 16 grams of non-woven fabric to the carding rollers. At the FBK unit, the fibers 320 are pushed down to a stainless steel plate and into the kiss roller machine, which comprises of a plurality of carding rollers. The FBK unit may comprise an induced draft (ID) fan which pushes the loosened fibers 320 into the carding rollers of the kiss roller machine. In a preferred aspect, there are four carding rollers. The FBK unit may comprise a control panel that controls the FBK unit by determining how much fibers 320 to distribute across the plurality of carding rollers based on the desired grams per square meter of non-woven fabric, such as a grams per square meter (gsm) required by a customer. As such, the FBK unit is important for controlling the gsm of the non-woven fabric.

In various aspects and as illustrated in Figure 4, the method may further comprise the step of carding the fibers 320 (step 3000) using a carding machine. Carding is a mechanical process whereby clumps of fibers 320 are separated into individual fibers 320 by loosening or combing, thereby forming a coherent web. The loosening or combing is controlled by selecting the speed of a plurality of rollers that loosen or comb the fibers 320 to form the coherent web.

In various aspects and as illustrated in Figure 4, the method may further comprise the step of passing through a conveyor belt (step 4000) followed by passing through an embossed roller and a smooth roller (step 5000). Advantageously, the conveyor belt serves to guide the coherent web through the embossed roller and the smooth roller. The embossed roller and the smooth roller may be filled with thermal oil and heated up to a required temperature to melt the coherent web so as to form the non-woven fabric i.e. first layer 20. In some aspects, the required temperature is about 150°C to about 180°C. In some aspects, the step of thermal bonding to form the first layer occurs at a temperature of 150°C to 180°C.

In various aspects and as illustrated in Figure 4, the method may further comprise the step of passing through a jumbo roll rewinding unit (step 6000), so that the non-woven fabric is wound to form a roll.

In various aspects and as illustrated in Figure 4, the method may further comprise the step of transporting the roll from step 5000 to a slitting machine so that the non-woven fabric may be slit to a desired dimension, such as a dimension required by a customer (step 7000).

Advantageously, the first layer 20 may have zero or negligible lint. Linting may occur as fibers 320 are pulled off or released from the surface of the first layer 20 and may result in fibers 320 undesirably remaining on the skin of the user. As such, use of the absorbent material 10 may be relatively more comfortable compared to currently known absorbent materials because the fibers 320 of the first layer 20 are thermally bonded to each other and then carded, thereby eliminating or minimizing linting.

In various aspects, the step of arranging the second layer 30 adjacent to the first layer 20 comprises the step of laminating the second layer 30 to the first layer 20. The step of laminating may make use of heat, mechanical or chemical means so that the interface between the second layer 30 and the first layer 20 is not visible i.e. the first layer 20 and the second layer 30 are substantially integrated or bonded such that it is difficult if not impossible to separate the two layers from each other, particularly when in use.

## Claims

1. An absorbent material (10) consisting of two layers:
a first layer (20) comprising a hydrophilic non-woven fabric; and
a second layer (30) comprising a hydrophobic film, the second layer laminated adjacent to the first layer such that the first and second layers are substantially integrated or bonded;
wherein the first layer is breathable and allows gas and liquid to pass through;
wherein the second layer is breathable and allows gas to pass through and minimizes or blocks liquid from passing through; and wherein the first layer comprises short fibers having an average length of 30 mm to 60 mm.

2. The absorbent material of claim 1, wherein the hydrophilic non-woven fabric comprises a polymer, optionally wherein the polymer is polypropylene or polyethylene.

3. The absorbent material of claim 1, wherein the hydrophobic film comprises polyethylene.

4. The absorbent material of any one of the preceding claims, wherein the thickness of the absorbent material is 0.1 millimetres (mm) to 5 mm.

5. The absorbent material of any one of the preceding claims, wherein the first layer is made using a thermal bonding process.

6. The absorbent material of claim 1, wherein the short fibers have an average length of 38 mm to 51 mm.

7. The absorbent material of claim 1, further comprising a super absorbent polymer, optionally wherein the super absorbent polymer is integrated with the first layer or integrated with the second layer.

8. The absorbent material of claim 1, wherein the first layer or the second layer is added with biodegradable additives.

9. A bed sheet made of an absorbent material according to any one of the previous claims.

10. A method of manufacturing an absorbent material, the method comprising:
the step of thermal bonding to form a first layer comprising a hydrophilic non-woven fabric;
the step of spin finishing prior to the step of thermal bonding;
the step of laminating a second layer comprising a hydrophobic film adjacent to the first layer such that the first and second layers are substantially integrated or bonded;
wherein the first layer is breathable and allows gas and liquid to pass through;
wherein the second layer breathable and allows gas to pass through and minimizes or blocks liquid from passing through; wherein the first layer comprises short fibers having an average length of 30 mm to 60 mm;
and wherein the step of thermal bonding comprises the step of carding.

11. The method of claim 10, wherein the step of laminating the second layer adjacent to the first layer comprises heat, mechanical or chemical means.

12. The method of claim 10 or 11, wherein the step of thermal bonding to form the first layer occurs at a temperature of 150°C to 180°C.

13. The method of any one of claims 10 to 12, further comprising the step of manufacturing fibers for the hydrophilic non-woven fabric.

14. The method of claim 13, wherein the step of manufacturing fibers for the hydrophilic non-woven fabric comprises the step of crimping, optionally wherein the step of crimping occurs at a pressure of 0.1 bar to 5.0 bar, optionally wherein the step of crimping occurs at a temperature of 60°C to 120°C.

## Patentansprüche

1. Absorbierendes Material (10), das aus zwei Schichten besteht:
einer ersten Schicht (20), die einen hydrophilen Vliesstoff umfasst; und
einer zweiten Schicht (30), die eine hydrophobe Folie umfasst, wobei die zweite Schicht benachbart zur ersten Schicht so laminiert ist, dass die erste und die zweite Schicht im Wesentlichen einstückig oder gebondet sind;
wobei die erste Schicht atmungsaktiv ist und ermöglicht, dass Gas und Flüssigkeit hindurchtreten;
wobei die zweite Schicht atmungsaktiv ist und ermöglicht, dass Gas hindurchtritt, und das Hindurchtreten von Flüssigkeit minimiert oder blockiert; und wobei die erste Schicht kurze Fasern umfasst, die eine mittlere Länge von 30 mm bis 60 mm aufweisen.

2. Absorbierendes Material nach Anspruch 1, wobei der hydrophile Vliesstoff ein Polymer umfasst, wobei das Polymer gegebenenfalls Polypropylen oder Polyethylen ist.

3. Absorbierendes Material nach Anspruch 1, wobei die hydrophobe Folie Polyethylen umfasst.

4. Absorbierendes Material nach einem der vorangegangenen Ansprüche, wobei die Dicke des absorbierenden Materials 0,1 Millimeter (mm) bis 5 mm beträgt.

5. Absorbierendes Material nach einem der vorangegangenen Ansprüche, wobei die erste Schicht unter Verwendung eines thermischen Bondingverfahrens hergestellt wird.

6. Absorbierendes Material nach Anspruch 1, wobei die kurzen Fasern eine mittlere Länge von 38 mm bis 51 mm aufweisen.

7. Absorbierendes Material nach Anspruch 1, das ferner ein superabsorbierendes Polymer umfasst, wobei das superabsorbierende Polymer gegebenenfalls mit der ersten Schicht oder mit der zweiten Schicht einstückig ist.

8. Absorbierendes Material nach Anspruch 1, wobei die erste Schicht oder die zweite Schicht mit biologisch abbaubaren Additiven hinzugefügt wird.

9. Bettlaken aus absorbierendem Material nach einem der vorangegangenen Ansprüche.

10. Verfahren zur Herstellung eines absorbierenden Materials, wobei das Verfahren Folgendes umfasst:
den Schritt des thermischen Bondens, um eine erste Schicht, umfassend einen hydrophilen Vliesstoff, auszubilden;
den Schritt des Spinnveredelns vor dem Schritt des thermischen Bondens;
den Schritt des Laminierens einer zweiten Schicht, umfassend eine hydrophobe Folie, benachbart zu der ersten Schicht, sodass die erste und die zweite Schicht im Wesentlichen einstückig oder gebondet sind;
wobei die erste Schicht atmungsaktiv ist und ermöglicht, dass Gas und Flüssigkeit hindurchtreten;
wobei die zweite Schicht atmungsaktiv ist und ermöglicht, dass Gas hindurchtritt, und das Hindurchtreten von Flüssigkeit minimiert oder blockiert; wobei die erste Schicht kurze Fasern umfasst, die eine mittlere Länge von 30 mm bis 60 mm aufweisen;
und wobei der Schritt des thermischen Bondens den Schritt des Kardierens umfasst.

11. Verfahren nach Anspruch 10, wobei der Schritt des Laminierens der zweiten Schicht benachbart zur ersten Schicht Hitze-, mechanische oder chemische Mittel umfasst.

12. Verfahren nach Anspruch 10 oder 11, wobei der Schritt des thermischen Bondens zum Ausbilden der ersten Schicht bei einer Temperatur von 150 °C bis 180 °C erfolgt.

13. Verfahren nach einem der Ansprüche 10 bis 12, das ferner den Schritt des Herstellens von Fasern für den hydrophilen Vliesstoff umfasst.

14. Verfahren nach Anspruch 13, wobei der Schritt des Herstellens von Fasern für den hydrophilen Vliesstoff den Schritt des Crimpens umfasst, wobei der Schritt des Crimpens gegebenenfalls bei einem Druck von 0,1 bar bis 5,0 bar durchgeführt wird, wobei der Schritt des Crimpens gegebenenfalls bei einer Temperatur von 60 °C bis 120 °C durchgeführt wird.

## Revendications

1. Matériau absorbant (10) constitué de deux couches :
une première couche (20) comprenant un tissu non tissé hydrophile ; et
une seconde couche (30) comprenant un film hydrophobe, la seconde couche étant stratifiée adjacente à la première couche de telle sorte que les première et seconde couches soient sensiblement intégrées ou liées ;
dans lequel la première couche est respirante et permet à du gaz et à du liquide de la traverser ;
dans lequel la seconde couche est respirante et permet à du gaz de la traverser et minimise ou empêche du liquide de la traverser ; et dans lequel la première couche comprend des fibres courtes présentant une longueur moyenne de 30 mm à 60 mm.

2. Matériau absorbant selon la revendication 1, dans lequel le tissu non tissé hydrophile comprend un polymère, facultativement dans lequel le polymère est du polypropylène ou du polyéthylène.

3. Matériau absorbant selon la revendication 1, dans lequel le film hydrophobe comprend du polyéthylène.

4. Matériau absorbant selon l'une quelconque des revendications précédentes, dans lequel l'épaisseur du matériau absorbant est de 0,1 millimètre (mm) à 5 mm.

5. Matériau absorbant selon l'une quelconque des revendications précédentes, dans lequel la première couche est fabriquée à l'aide d'un procédé de liaison thermique.

6. Matériau absorbant selon la revendication 1, dans lequel les fibres courtes présentent une longueur moyenne de 38 mm à 51 **mm.**

7. Matériau absorbant selon la revendication 1, comprenant en outre un polymère superabsorbant, facultativement dans lequel le polymère superabsorbant est intégré à la première couche ou intégré à la seconde couche.

8. Matériau absorbant selon la revendication 1, dans lequel des additifs biodégradables sont ajoutés à la première couche ou la seconde couche.

9. Drap de lit en matériau absorbant selon l'une quelconque des revendications précédentes.

10. Procédé de fabrication d'un matériau absorbant, le procédé comprenant :
l'étape de liaison thermique pour former une première couche comprenant un tissu non tissé hydrophile ;
l'étape de finition par centrifugation avant l'étape de liaison thermique ;
l'étape de stratification d'une seconde couche comprenant un film hydrophobe adjacent à la première couche de telle sorte que les première et seconde couches soient sensiblement intégrées ou liées ;
dans lequel la première couche est respirante et permet à du gaz et à du liquide de la traverser ;
dans lequel la seconde couche est respirante et permet à du gaz de la traverser et minimise ou empêche du liquide de la traverser ; dans lequel la première couche comprend des fibres courtes présentant une longueur moyenne de 30 mm à 60 mm ;
et dans lequel l'étape de liaison thermique comprend l'étape de cardage.

11. Procédé selon la revendication 10, dans lequel l'étape de stratification de la seconde couche adjacente à la première couche comprend des moyens thermiques, mécaniques ou chimiques.

12. Procédé selon la revendication 10 ou 11, dans lequel l'étape de liaison thermique pour former la première couche survient à une température de 150°C à 180°C.

13. Procédé selon l'une quelconque des revendications 10 à 12, comprenant en outre l'étape consistant à fabriquer des fibres pour le tissu non tissé hydrophile.

14. Procédé selon la revendication 13, dans lequel l'étape consistant à fabriquer des fibres pour le tissu non tissé hydrophile comprend l'étape de crêpage, facultativement dans lequel l'étape de crêpage survient à une pression de 0,1 bar à 5,0 bars, facultativement dans lequel l'étape de crêpage survient à une température de 60°C à 120°C.
